Europäisches Patentamt

European Patent Office

Office européen des brevets

(11) Publication number: 0 383 183
A1

# (12) EUROPEAN PATENT APPLICATION

(21) Application number: 90102424.0

(22) Date of filing: 07.02.90

(51) Int. Cl.⁵: C12N 15/00, C12P 21/08

(30) Priority: 08.02.89 JP 29313/89

(43) Date of publication of application:
22.08.90 Bulletin 90/34

(84) Designated Contracting States:
DE FR GB

(71) Applicant: OLYMPUS OPTICAL CO., LTD.
43-2, 2-chome, Hatagaya Shibuya-ku
Tokyo 151(JP)

(72) Inventor: Kano, Kyoichi
1-4-33-702, Takanawa, Minato-ku
Tokyo(JP)
Inventor: Takiguchi, Masafumi
6-3-4 Minamisenju,Arakawa
Tokyo(JP)

(74) Representative: Kuhnen, Wacker & Partner
Schneggstrasse 3-5 Postfach 1553
D-8050 Freising(DE)

(54) A method for producing anti-HLA monoclonal antibodies.

(57) Steps of (a) introducing HLA gene into a fertilized egg or embryo of a non-human mammal, (b) transferring the fertilized egg or the embryo of the non-human mammal obtained in the step of (a) into a uterus of a female non-human mammal which is a foster mother, (c) breeding the female non-human mammal of a foster mother for a given period so that the mammal may reproduce transgenic non-human mammals, (d) administering anti-HLA antigen as immunogen into the transgenic non-human mammals which are obtained in the step of (c) and whose HLA genes introduced are expressed, (e) enucleating the spleen from the transgenic non-human mammals obtained in the step of (d) and making lymphocytes obtained from the spleen and myeloma cells to fuse, (f) selecting hybridomas which produce anti-HLA antibodies among the hybridomas obtained in the step of (e), and (g) culturing the selected hybridomas in vivo or in vitro to obtain anti-HLA monoclonal antibodies are comprised. In place of the above described steps of (a)-(c), steps of (a) transferring embryonal stem cells to which HLA genes are introduced into uterus of female non-human mammal of a foster mother simultaneously with an embryo of non-human mammal, (b) breeding the female non-human mammal of a foster mother so that the mammal may reproduce chimera individuals having HLA genes in their germ cells, and (c) performing a passage of the chimera individuals obtained in the step (b) to obtain transgenic non-human mammals having HLA genes in their all germ cells and somatic cells may also be conduced, and the steps on and after the above described step (d) may be similarly performed by utilizing this transgenic non-human mammal.

```
EXON-2(α1-DOMAIN).                                    50
B51    GCTCCCACTCCATGAGGTATTTCTACACCGCCATGTCCCGGCCCGGCCGC
BW52   -------------------------------------------------

                                                      100
B51    GGGGAGCCCCGCTTCATTGCAGTGGGCTACGTGGACGACACCCAGTTCGT
BW52   ------------------------C------------------------

                                                      150
B51    GAGGTTCGACAGCGACGCCGCGAGTCCGAGGACGGAGCCCCGGGCGCCAT
BW52   -------------------------------------------------

                                                      200
B51    GGATAGAGCAGGAGGGGCCGGAGTATTGGGACCGGAACACACAGATCTTC
BW52   ---------------------------------G-G---------C-

                                                      250
B51    AAGACCAACACACAGACTTACCGAGAGAACCTGCGGATCGCGCTCCGCTA
BW52   -------------------------------------------------

                 270
B51    CTACAACCAGAGCGAGGCCG
BW52   --------------------
```

F I G. 1B

## A method for producing anti-HLA monoclonal antibodies

This invention relates to a method for producing anti-HLA monoclonal antibodies, particularly to a method for producing the anti-HLA monoclonal antibodies by utilizing transgenic non-human mammals to which HLA genes are introduced.

HLA antigens play a significant part in transplantation of human organs through transplantation immunity, and function as antigens which determine whether or not transplanted organs or tissues are acceptable.

HLA antigens are called major histocompatibility antigens (MHA) for profoundly involved in rejection and transplantation immunity. Gene groups coding for these antigens are accordingly called major histocompatibility complex (MHC).

HLA are human MHA, and HLA class I antigens are membrane protein on cell membranes and controlled by three gene loci comprising HLA-A, HLA-B, and HLA-C. A class I antigen comprises glycoprotein (alpha-chain) of a 44 kd molecular weight associated with protein ($\beta$2-microglobulin) of a 12 kd molecular weight through non-covalent bond. The alpha-chains are composed of three domains such as $\alpha$1, $\alpha$2, and $\alpha$3 at extracellular N-terminus site, followed by transmembrane region which is embedded in cell membranes and intracytoplasmic domain which locates in the cell. The $\beta$2-microglobulin is composed of one domain and combined with the alpha-chain.

A HLA class II antigen is a heterodimer comprising an alpha-chain of membrane-penetrating glycoprotein of a 35 kd molecular weight bonded with a beta-chain of a 27 kd molecular weight through non-covalent bond. As the class II antigens, DP antigen, DQ antigen and DR antigen are known. These antigens are found to be slightly different in their molecule sizes of the chains constituting themselves.

HLA gene region is present on short-arm of human sixth chromosome over 2.5 centimorgan (cM) having from a centromere site class II region (1.0 cM), class III region (DR-B distance = 0.7 cM), and class I region (0.8 cM) in this order (Jordan, B.R. et al. Immunol. Rev., 84, 73, 1985).

One of methods for detecting HLA class I antigens is serological method. The typical serological method is complement-dependent cytotoxicity test (Terasaki, P.I. and McClelland, J.D. Nature (London), 204, pp 998-1000, 1964). This method introduces alloantiserum into a sample and types HLA by means of lymphocyte cytotoxicity utilizing rabbit's serum as complement. In this method, however, the antiserum must be alloserum of a grand multipara. In addition, since the antiserum shows cross reaction, antiserum which specifically react with HLA class I allotype cannot be easily obtained. Since antiserum is obtained from a human body, stable supply of serum with reproducibility cannot be attained. Furthermore, antiserum against to alloantigen with a low degree of expression cannot be obtained. Under the circumstances, monoclonal antibodies which replace the alloserum are thus expected to be produced. Approximately 150 kinds of monoclonal antibodies have been introduced in The International Histocompatibility Workshop in 1984 (Fauchet, R., Bonder, J.G., Kennedy, L.J. et al, : HLA-A, B, C monoclonal antibodies. Histocompatibility Testing 1984 (Albert, E.D., Baur, M.P., Mayr, W.R. ed), Springer-Verlag, Berlin, Heidelberg, New York, Tokyo: 211, 1984).

Anti-HLA monoclonal antibodies are generally produced by a method which fuses mouse splenocyte derived from a mouse immuned by human lymphocytes and mouse myeloma cells (Oi, V.T., Herzenberg, L.A.: Immunoglobulin-producing hybrid cell lines. Selected method in Immunology, 351, 1981). However, among monoclonal antibodies produced up to date, antibodies suitable for HLA typing is quite few. For example, antibodies against allotypes of HLA-B antigens include monoclonal antibodies which specifically conjugate with Bw4, Bw6, B7, B8, B13, and B27 only. Therefore, monoclonal antibodies having specificity with other many allotypes are expected to be developed.

At present, there are many alloantigens, with respect to which monoclonal antibodies having reaction have not been obtained. For such alloantigens, monoclonal antibodies having cross reaction with other alloantigens are effective.

Recently an improved method has been developed for producing anti-HLA monoclonal antibodies. In conventional method for producing antibodies utilizing human T lymphocyte-dominant peripheral blood lymphocytes as immunogen, since many antigens derived from human beings are inconveniently present, monoclonal antibodies which are reactive with many epitopes other than objective HLA allotype antigens are obtained. The improved method circumvents in this respect over the conventional method by introducing human HLA antigen gene clone into eucaryocytes derived from animals for immunity to obtain transformed cells, and by administering said transformed cells to said animals to obtain splenocytes which efficiently produce monoclonal antibodies against human HLA antigens expressed on the transformed cells (Monoclonal antibodies to HLA-DP-transfected mouse L cell: Proc. Natl. Acad. Sci., USA, 83, pp 3417-3421, 1986). However, for an immuned animal such as a mouse, species-specific antigen determinants (isotype) are surprisingly strong in antigenicity which are not recognized between human individuals rather than slightly different antigen determinants (allotype) which are recognized between human individuals. Therefore, this method inconveniently produces monoclonal antibodies against isotypes, namely, those cross-reacting with a plural of alloantigens are produced in a large amount.

On the other hand, transgenic animals are produced by introducing foreign genes. Specifically, a mouse is often utilized for its easy manipulation in experiments and easy availability, and many transgenic mice have been produced. Among several features of transgenic animals, two of them are typically described as follows. First, introduced genes are integrated in normal cells. Second, since a fertilized egg to which a gene is introduced develops and differentiates to a single individual, the foreign genes are present in the same locus of chromosomes of all cells constituting the individual.

Transgenic mice are reportedly utilized for the following studies;

a) Relation Analysis between Gene Constructions and Expression. (Kondoh, H. et al., Dev. Biol., 120, pp 177-185, 1987; brinster, R.L. et al., Nature, 3, pp 332-336, 1983)

b) Production of Human Disease Model Mouse (Wakasugi S. et al., A transgenic mouse model of familial amyloid polyneuropathy. Proc Jpn-Acad, 63, 344, 1987; Leder et al., TRANSGENIC NON-HUMAN MAMMALS, United State Patent No. 4,736,866 (1988))

c) Analysis of Gene Product Roles Intracellularly or in Individuals. In immunity, transgenic mouse is utilized in analysis of roles of MHC class I antigens or class II antigens. (Frels, W. I. et al., Science, 228, 577, 1985; Le Meur. M. et al., Nature, 316, 38, 1985; Yamamura K. et al., Nature, 316, 67, 1985; Femia Kievits et al., Nature, 329, pp 447-449, 1987).

However, transgenic animals have not been reportedly utilized for monoclonal antibody production up to date.

The object of the present invention is to provide a method for efficiently produce monoclonal antibodies against antigen determinants showing slight differences which can be recognized between human individuals only.

This object can be attained by the following steps of;

a) introducing HLA gene into a fertilized egg or an embryo of non-human mammals,

b) transferring said fertilized egg or the embryo of the non-human mammals obtained in the step of (a) into uterus of a female non-human mammal which is a foster mother,

c) breeding the female non-human mammal of a foster mother for a given period so that the mammal may reproduce transgenic non-human mammals,

d) administering HLA antigens as immunogen into said transgenic non-human mammals whose introduced HLA genes are expressed,

e) enucleating a spleen from the transgenic non-human mammal immuned in the step of (d) and making lymphocytes obtained from the spleen and myeloma cells to fuse,

f) selecting hybridomas which produce anti-HLA antibodies among hybridomas obtained in the step of (e), and

g) culturing the selected hybridomas in vivo or in vitro to obtain said anti-HLA monoclonal antibodies.

Said object can be also attained by the following steps:

a) transferring embryonal stem cells having HLA gene introduced into a uterus of female non-human mammal of a foster mother simultaneously with an embryo of a non-human mammal,

b) breeding the female non-human mammal of a foster mother for a given period so that the mammal reproduce chimera individuals having HLA genes in their germ cells,

c) performing a passage of said chimera individuals obtained in the step of (b) to obtain transgenic non-human mammals having HLA genes in their all germ cells and somatic cells,

d) administering HLA antigens as immunogen into transgenic non-human mammals which are obtained in the step of (c) and whose HLA genes introduced are expressed,

e) enucleating a spleen from the immuned transgenic non-human mammal and making lymphocytes obtained from the spleen and myeloma cells to fuse,

f) selecting hybridomas which produce anti-HLA antibodies among hybridomas obtained in the step of (e), and

g) culturing said hybridomas selected in the step of (f) in vivo or in vitro to obtain anti-HLA monoclonal antibodies.

Additional objects and advantages of the invention will be set forth in the description which follows, and in part will be obvious from the description, or may be learned by practice of the invention. The objects and advantages of the invention may be realized and obtained by means of the instrumentalities and combinations particularly pointed out in the appended claims.

This invention can be more fully understood from the following detailed description when taken in conjunction with the accompanying drawings, in which:

The accompanying drawings, which are incorporated in and constitute a part of the specification, illustrate presently preferred embodiments of the invention, and together with the general description given above and the detailed description of the preferred embodiments given below, serve to explain the principles of the invention.

Figs. 1A-1G show complete sequences of HLA-B51 and HLA-Bw52;

Fig. 2 is a photograph showing an autoradiography positive pattern obtained in Dot Blot Hybridization of B51-16-1 mouse according to an embodiment of the present invention;

Fig. 3A is a graph showing results of a test for confirming expression of HLA-B51 antigen for lymphocytes of normal C3H mouse in one example of the present invention;

Fig. 3B is a graph showing results of a test for confirming expression of HLA-B51 antigen for lymphocytes of B51-16-1 mouse in one example of the present invention; and

Figs. 4A-4G are graphs showing results of flow cytometry for B-51-16-1-4, B-51-16-1-7, B51-16-1-8, B51-16-1-9, B51-16-1-11, B51-16-1-12, and B51-16-1-16 mouse respectively, in one example of the present invention.

According to the present invention, a method for producing anti-HLA monoclonal antibodies utilizese transgenic non-human mammals to which HLA genes are introduced as animals for immuning. These non-human mammals to which HLA genes are introduced include primates such as rhesuses and chimpanzees, and Rodent such as mice and rats.

Transgenic non-human mammals may be obtained by introducing HLA genes into a fertilized egg or embryo of a non-human mammal, transferring the fertilized egg or the embryo into uterus of the foster mother, and breeding the mammal for a given period so that the mother may reproduce individuals.

Instead of above-mentioned method, transgenic non-human mammals can also be obtained by transferring embryonal stem cells to which HLA genes are introduced into uterus of a foster mother with an embryo of a non-human mammal, so that the foster mother may reproduce chimera individuals. However, although the chimera individuals thus obtained have HLA genes in germ cells, they have no HLA genes in somatic cells. Therefore, a passage of the chimera individuals are performed such that they contain HLA genes in both germ cells and somatic cells. Transgenic non-human mammals can thus be obtained from the following generation.

In order to introduce HLA genes into a fertilized egg, an embryo or embryonal stem cells, known methods can be utilized. The examples of the methods include (1) isolated HLA genes are directly injected into cells utilizing micromanipulators, (2) Recombinant retro viruses to which HLA genes are introduced are infected in cells, and (3) HLA genes are incorporated into cells after treating CaCl2 of high concentration.

Transgenic non-human mammals thus obtained contain HLA genes in all germ cells and somatic cells, and all eucaryocytes or a part of them (e.g. T-cell) express HLA antigens which are coded by introduced HLA genes.

Into these transgenic non-human mammals, HLA antigens are administered as immunogen to immunize the mammals. The immunogen is preferably HLA alloantigen which is different from alloantigen which is coded by the HLA gene introduced into mammals that are to be administered. The immunogen may be administered in the form of eucaryocytes which express HLA antigens or lysates thereof.

Next, a spleen is enucleated from the immuned animal, and lymphocytes obtained from the spleen is fused with myeloma cells to obtain hybridomas which produce objective anti-HLA antibodies. The hybridomas are cultured in vivo or in vitro to obtain anti-HLA monoclonal antibodies. To obtain high titer antibodies, the hybridomas are preferably in vivo cultured by introducing into mouse infraperitoneally. Hybridomas may also be in vitro cultured in a medium to easily obtain purified monoclonal antibodies.

HLA antigens to be introduced to a fertilized egg, an embryo or embryonal stem cells is determined by the objective monoclonal antibody. For example, when monoclonal antibodies against HLA class I allotype are desired to obtain, HLA class I genes are selected which code alloantigens that are different from HLA class I alloantigens which are specifically combined with the objective monoclonal antibodies. Another example is HLA class II gene. The HLA genes, which are introduced into non-human mammals in order to obtain transgenic non-human mammals, and HLA antigens, which function as immunogens administrated to immune the transgenic non-human mammals, are used to develop monoclonal antibodies with desired specificity. For example, a combination of alloantigens belonging to cross reactive groups (CREG) exhibiting cross reaction in the conventional serological assay method is selected thereby developing an antibody capable of recognizing an allospecific epitope, which has not been realized by the conventional serological method. The following are typical examples of the combinations of an HLA gene for coding an alloantigen belonging to the CREG, which is used as a gene to be introduced, and another alloantigen belonging to the CREG, which is as an immunogen:
(A2, A28, A27), (A2, A24, A26), (A3, A11), (A24, A32), (B35, Bw53), (B51, Bw52), (B8, B51, Bw52), (B27.1, B27.2, B27.3, B27.4), (B40, Bw41), (B13, B51), (B51, Bw52, B35), (B51, B8), (Bw60, B40), (Bw4, Bw6), (B7, B27), (B14, B65), (B38, B39, B58, B51, B52), (Cb-1, Cb-2), (Cw2.1, Cw2.2), etc.

In the above-listed examples, one CREG is expressed in parentheses (). In each pair of parentheses, a character, e.g. A2, denotes HLA-A2 gene or HLA-A2 antigen. Thus, for example, the CREG (B51, Bw52, B35) means that this CREG is composed of HLA-B51, HLA-Bw52, and HLA-B35, and two (a gene and an antigen) of HLA-B51, HLA-B5 and HLA-B35 can be combined. Moreover, genes introduced into cells need not be of one kind, and several HLA genes for coding a plural of alloantigens may be introduced. By introducing a plural of HLA genes, reactivity of transgenic non-human mammals against foreign bodies can be improved nearly to human beings.

Detailed description of the present invention will be described with reference to the following examples.

## Production of HLA-B51 Transgenic Mouse

### A. Purification of HLA-B51 Gene

Thirty microgram of plasmid pHLA-B51 which is pBR328 including HLA-B51 gene of 6.4 kb at EcoRI site were made to react with 90 units of EcoRI at $37^\circ$C for 60 minutes to cleave EcoRi sites. The resultant cleaved fragments were deposited to the end region of an agarose gel for electrophoresis. Next, a band of 6.4 kb were cut off with a knife to separate HLA-B51 genes. The separated genes were put into a 1.5 m$\ell$-test tube, and a ca. 2-fold sodium iodide solution was added, followed by dissolution in a water bath at $55^\circ$C. Next, 5-10 $\mu\ell$ of silica matrix (GIASSMILK, manufactured by GENECLEAN$^{TH}$ CO.) was added to the test tube for mixing, and the resultant solution was incubated at $4^\circ$C overnight. The test tube was then centrifuged at 15000 rpm for five minutes to remove a supernatant solution. After that, to the tube, 0.2 m$\ell$ of washing solution containing 10 mM Tris pH 7.5, 1 mM EDTA, 0.1M NaC$\ell$ and 50% ethanol was added, and the mixture was stirred with a pipette, followed by centrifugation at 15000 rpm for five minutes. This washing operation was repeated for three times. After these washing operations and exclusions of supernatant solutions, 50 mM Tris, pH 9.0, and 20 $\mu\ell$ of 0.2M NaC$\ell$ solution were added to the tube, and the resultant mixture was incubated at $60^\circ$C for 30 minutes. The tube was centrifuged at 15000 rpm for 30 seconds, and the obtained supernatant solution was poured into a new test tube. To the new tube, 50 mM Tris, pH 9.0, and 20 $\mu\ell$ of 0.2M NaC$\ell$ solution were added, and the mixture was incubated at $60^\circ$C for 30 minutes. After this incubation, the tube was further centrifuged at 15000 rpm for 30 minutes to separate a supernatant solution. To the supernatant solution, ethanol was added for DNA precipitation. The obtained DNA precipitates were dried and then dissolved in a solution containing 10 mM Tris, pH 7.59, and 0.5 mM EDTA to prepare a DNA solution of 500 copies/pl.

### B. Picking of a Fertilized Egg

To C3H female mice (C3H/He, obtained from SHIZUOKA ANIMAL CENTER), 5 units of pregnantmare serum (PMS) was intraperitoneally injected, and 5 units of chorionic gonadotropin (HCG) was further injected after 48 hours. These mice were put into a cage containing C3H male mice. The mice were observed overnight to confirm copulation. In the following morning, female mice which were confirmed to have copulation were killed, and their oviducts and parts of uteruses were enucleated. These enucleated organs were put in a Petri dish including a medium containing 1 mg/m$\ell$ of hyaluronidase, and the ampulla tubae uterinae was cut open under a stereoscopic microscope to pick a fertilized egg. This fertilized egg was treated with a hyaluronidase, washed with medium for three times, and cultured in a $CO_2$-incubator at $37^\circ$C.

### C. Gene Induction

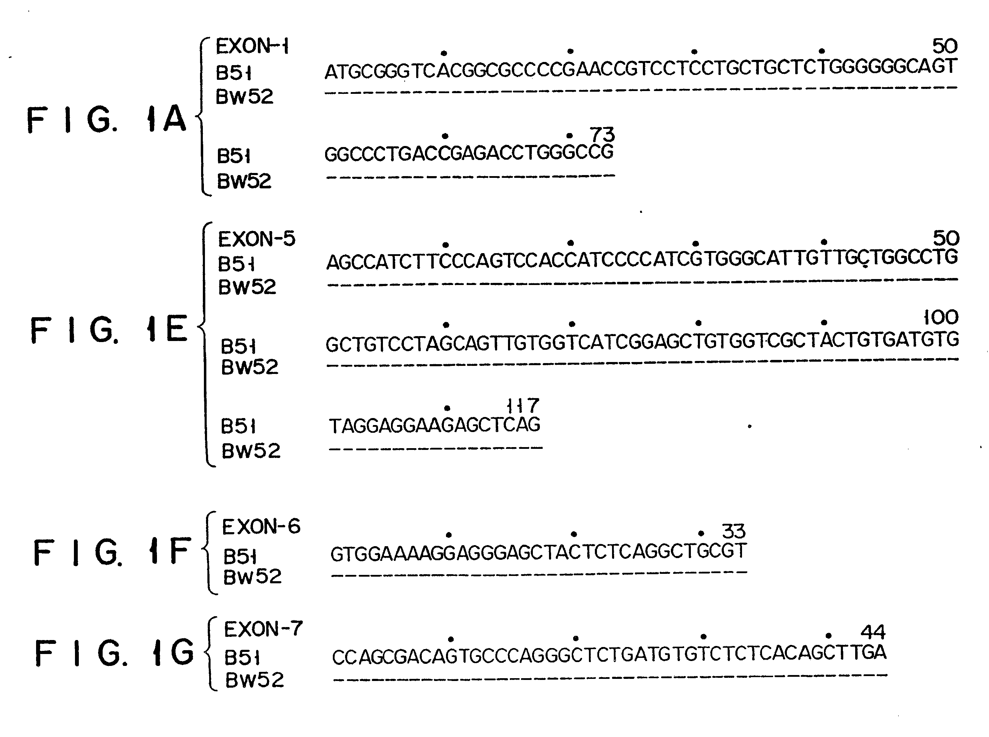

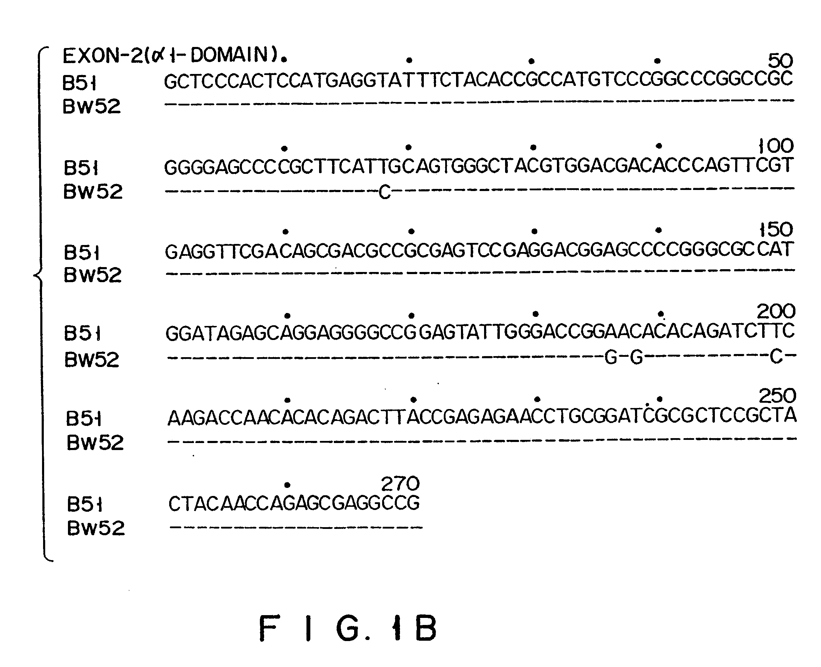

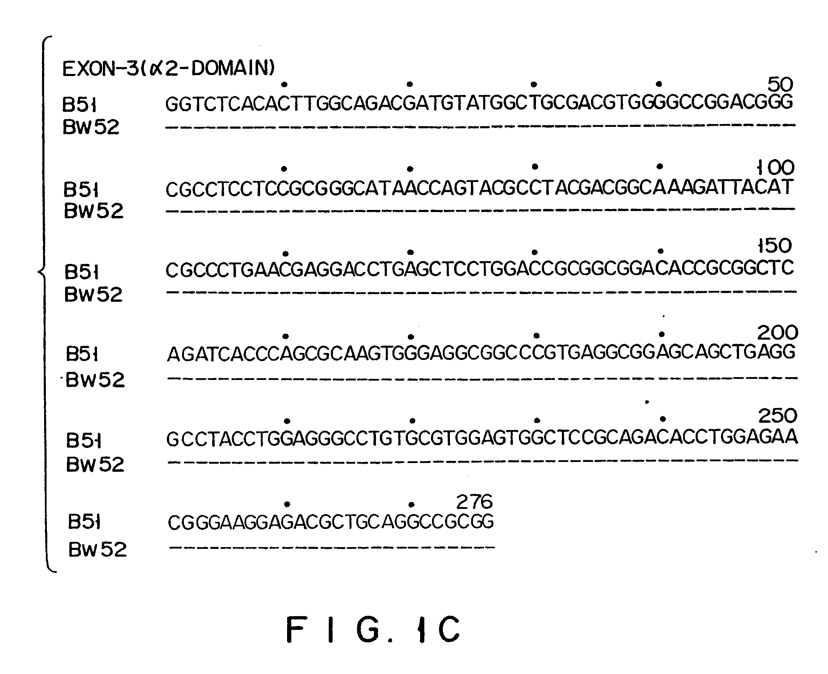

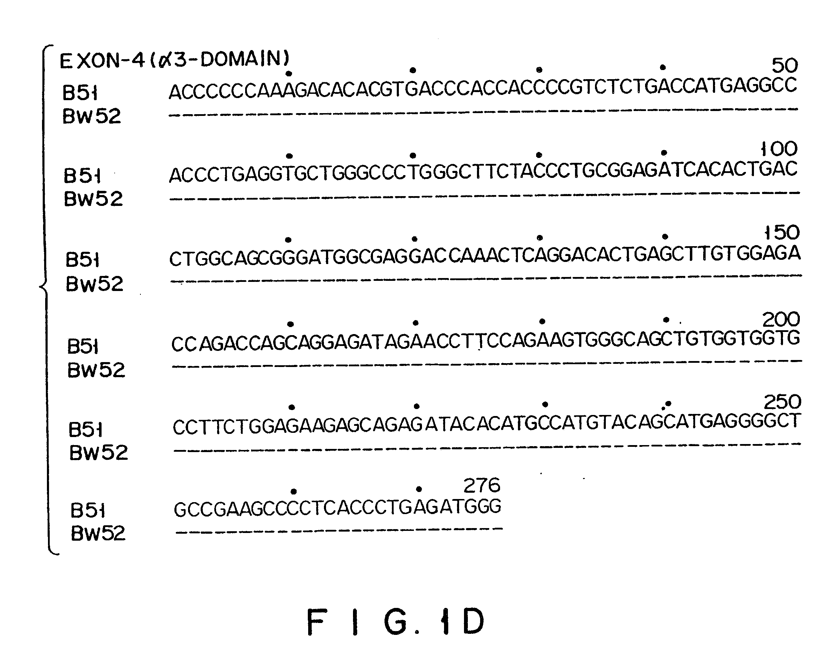

First, drops containing a medium and a solution of HLA-B51 genomic DNA having base sequences shown in Figs. 1A-1G were prepared in a culture dish, and paraffin oil was flowed thereon to cover the drops. Next, the fertilized egg as picked above was introduced into the drops of the culture solution. The resultant culture dish was then set in an injection microscope. This microscope is accompanied with a micromanipulator having a holding pipette for keeping a fertilized egg and an injection pipette for injecting a DNA solution. After setting the dish into the microscope, the DNA solution was aspirated. Next, with the fertilized egg fixed with the carrier pipette, the DNA solution in the injection pipette was injected into male pronuclei of the egg.

### D. Transfer of Gene-Introduced Embryo into a Foster Mother

First, a male mouse was made to be sterile by vasectomy. Next, this male mouse and a female ICR mouse were allowed to copulate to make the female mouse pseudopregnancy.

Next, to the pseudopregnant female mouse, the transgenic fertilized egg as obtained above was transferred in the following manners. First, 6 mg/mℓ of Nembutal was intraperitoneally injected to the mice at 0.3 mℓ/one mouse to anesthetize them. Each mouse was then made to lie on her face, and the skin and muscular layers were cut open from the back to pick and take out fatty masses around the overium with tweezers. Ostium abdominale was found in the fatty masses, and the transgenic fertilized egg was transferred into the uterine tube with transplanting pipettes. Then, the enucleated organs such as the ovarium, the uterine tube, the uterus, etc. were returned to the mouse, followed by suture of the muscular layers and fastening of the skin with clips. After that, mice which were reproduced in ca. 20 days were made to be brought up by ICR mice which already delivered.


E. Confirmation of Transgenic Mouse

In order to confirm that the objective genes are introduced into said obtained mice and that the genes are expressed, two methods are conducted as follows. Mice utilized for confirmation were of about four weeks.

1) The first method is to confirm HLA-B51 gene was incorporated into chromosomes of the mice by means of Dot Blot Method utilizing DNA derived from tails of the mice.

First, ca. 1 cm of a tail of a four week-aged mouse is cut off and is put into a 1.5 mℓ test tube. To this tube, 0.7 mℓ is SET buffer containing 5 mℓ of 1M Tris-HCℓ, pH 8.0, 2.5 mℓ of 20% SDS, and 42.5 mℓ of $H_2O$ and 20 μℓ of 10 mg/mℓ proteinase K are added, and the resultant mixture is shaken at 37°C overnight for dissolution. From this tube, 70 μℓ of the solution is transferred to a new test tube, and 140 μℓ of TE buffer containing 10 mM Tris-Cℓ and 1 mM EDTA, pH 8.0, are added for dilution. To this solution, 200 μℓ of phenol is further added, and the resultant solution is centrifuged at 15000 rpm for five minutes. After centrifugation, the resultant supernatant solution is transferred to a new test tube. To this tube, 20 μℓ of 3M sodium acetate and 500 μℓ of 99% ethanol are added, and the resultant solution is stirred, followed by incubation at -70°C for 15 minutes. After incubation, the solution is centrifuged at 4°C at 15000 rpm for 15 minutes, and the obtained supernatant solution is removed, followed by drying of the solution. After drying, 2M NaCℓ and 100 μℓ of 0.1N NaOH solution are added to the pellet obtained after drying. The resultant mixture is fully stirred for dissolution and incubated at 100°C for five minutes. After that, the obtained solution is rapidly quenched in ices to obtain a sample.

Apart from said operation, a nitrocellulose membrane of 15 cm × 12.5 cm is immersed in water for five minutes, and further immersed in 3-fold SSC buffer for five minutes. Next, Whatman 3MM filter paper which have also been immersed in 3-fold SSC buffer is mounted between a gum gasket and a sample plate of a Dot Blot machine (manufactured by Bio-Rad Company), and then said nitrocellulose membrane is mounted and fixed on the filter paper. The sample obtained above is introduced to this Dot Blot machine so that the sample is absorbed to the nitrocellulose membrane. After absorption, the nitrocellulose membrane is dismantled and then dried. Next, the dried membrane is kept in a vacuum dryer at 80°C for 2 hours to fix the DNA on the membrane.

The membrane having the DNA fixed is put into a hybridization bag, followed by addition of a pre-hybridization solution. Then the bag is sealed and incubated at 42°C for two hours. The pre-hybridization solution used contains 0.8 mℓ of 50-fold Denhardt's solution, 10 mℓ of formamide, 0.2 mℓ of 10% SDS, 0.5 mℓ of 4 mg/mℓ salmon sperm DNA, 6.6 mℓ of 20-fold SSC, and 1.9 mℓ of $H_2O$. After incubation, the pre-hybridization solution is removed from the bag, and a hybridization solution is added instead. The bag is then sealed and incubated at 42°C for 18 hours. The hybridization solution used is prepared as follows; $^{32}$P-dCTP labeled 1 × 10[17] cpm of HLA-B7cDNA probes and 0.5 mℓ of 4 mg/mℓ salmon sperm DNA are put into a glass test tube and heated in a water bath at 100°C for ten minutes, followed by rapid quenching in ices. To this tube, a mixture solution containing 0.8 mℓ of 50-fold Denhardt's solution, 10 mℓ of formamide, 0.2 mℓ of 10% SDS, 6.6 mℓ of 20-fold SSC solution, and 2 mℓ of 50% dextran sulfate is added to prepare the hybridization solution. After incubation, the nitrocellulose membrane is removed and washed with a washing solution containing 2-fold SSC and 0.1% SDS at 37°C for 30 minutes twice, followed by further washing at 52°C for 30 minutes twice. The membrane is then dried. Next, the dried membrane is fixed on Whatman 3MM filter paper, and set into a cassette of X-ray films, followed by exposure of the X-ray films for 3-18 hours and development of the films. When HLA-B51 gene is present in the sample, black dots appear on the films. Therefore, by these dots, it is confirmed that HLA-B51 gene is incorporated into the mouse chromosomes.

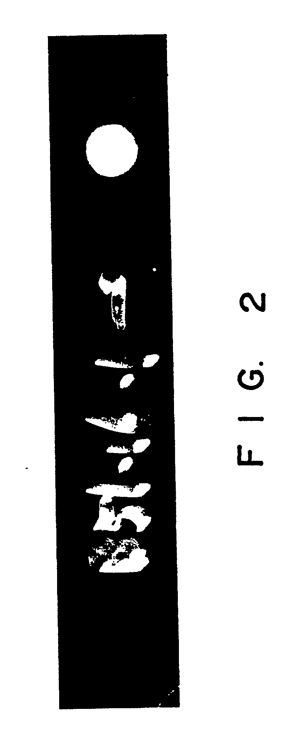

Forty-three mice in which genes were introduced by the A)-D) steps as said above were subjected to this first method to confirm incorporation. As a result, one of them (B51-16-1, female) was confirmed to have HLA-B51 gene incorporated. Fig. 2 shows a positive pattern of autoradiogram obtained from Dot Blot Hybridization of this B51-16-1 mouse.

2) The second method is to separate lymphocytes from the peripheral blood and to confirm that HLA-B51 alloantigen is expressed on the lymphocytes by utilizing anti-HLA class I antibodies.

First, 0.2 ml of peripheral blood from HLA-B51-transgenic mouse and 0.2 ml of peripheral blood from a C3H mouse as negative control are collected, and lymphocytes are separated by utilizing Ficoll-Paque. The obtained lymphocytes are then washed with RPMI medium three times, and then a lymphocyte suspension is prepared containing $5 \times 10^5$ lymphocytes in 50 $\mu$l of RPMI medium. This suspension is poured into a test tube such that the tube contain 50 $\mu$l of the suspension. A diluted FITC-labeled anti-HLA class I antibody W6/32 is further added to the tube. Then, the tube is incubated at 4°C for 30 minutes. After that, 1 ml of PBS is added to the tube, followed by centrifugation at 1500 rpm for five minutes and removal of the supernatant solution. This step is repeated three times such that 500 $\mu$l of the solution be finally obtained in the tube.

Expression of HLA-B51 gene can be confirmed by measuring fluorescence of the solution obtained. Namely, when the above obtained solution shows stronger fluorescence than lymphocytes of a control mouse, HLA-B51 antigens of human HLA antigens are confirmed to express on the lymphocytes. To measure fluorescence intensity, flowcytometer FACStar (manufactured by Bectondickinson Co.), for example, may be used.

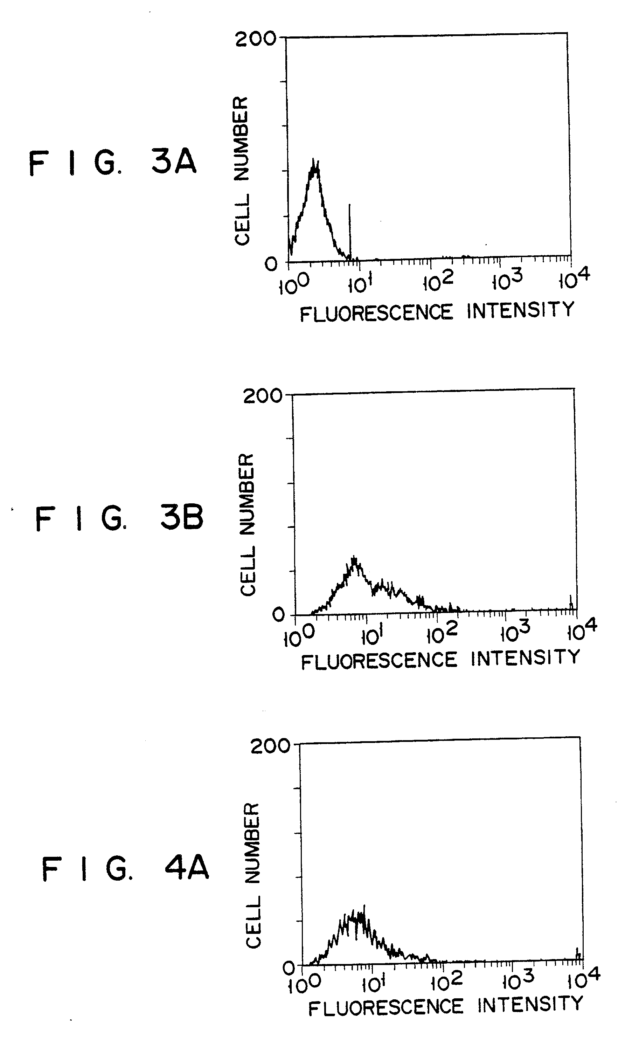

To 12 week-aged B51-16-1 transgenic mouse which show positive pattern in said hybridization of the first method, said second method was performed to confirm HLA-B51 antigens are expressed on the lymphocytes. The result is shown in Fig. 3B. As apparently from Fig. 3B, B51-16-1 transgenic mouse has HLA-B51 antigen expressed on the lymphocytes. For comparison, Fig. 3A shows negative pattern of lymphocytes of a 4 week-aged normal C3H mouse tested.

F. Establishment of a Transgenic Mouse

As described above, B51-16-1 mouse ($F_0$ mouse) is HLA-B51 transgenic, which is obtained from development of a micro-injected fertilized egg by said operation. To establish transgenic line, this B51-16-1 mouse (female) was made to cross with a congeneric mouse to reproduce.

Cross was conducted by putting B51-16-1 mouse (female) and normal C3H mouse (male) in the same cage. In about three weeks, the female mouse reproduced several mice ($F_1$). This process was repeated, as a result, the female mouse was impregnated three times and 16 mice were born. To the $F_1$ mice, said Dot Blot Hybridization was conducted, and said flow cytometry was further conducted to mice which show positive to said Dot blot Hybridization.

As a result of the Dot Blot Hybridization, seven mice of B51-16-1-4, B51-16-1-7, B51-16-1-8, B51-16-1-9, B51-16-1-11, B51-16-1-12, and B51-16-1-16 were found to have HLA-B51 gene incorporated.

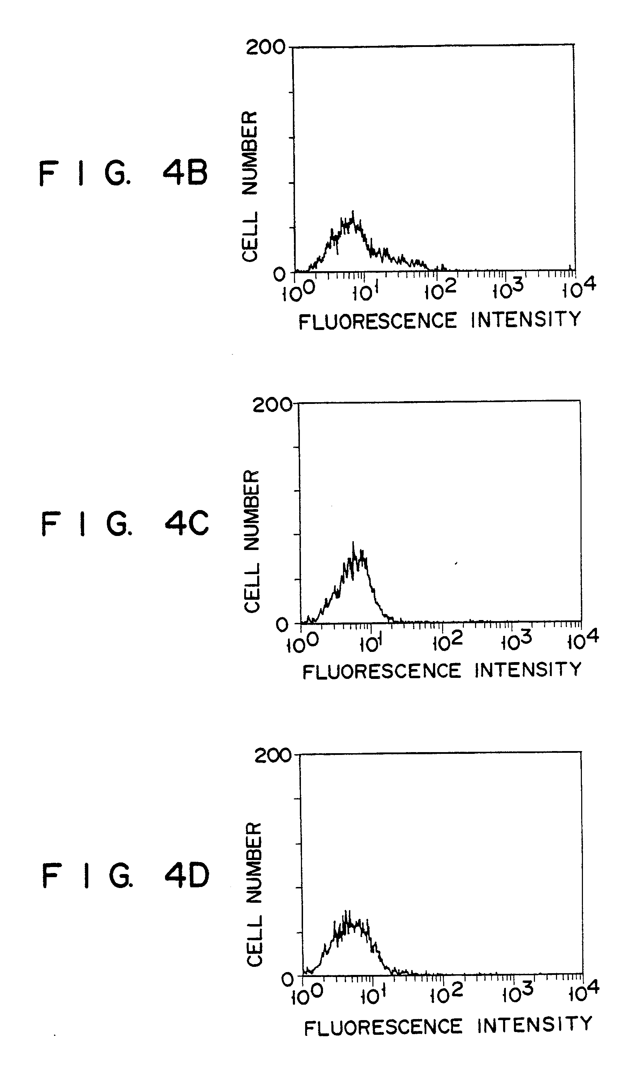

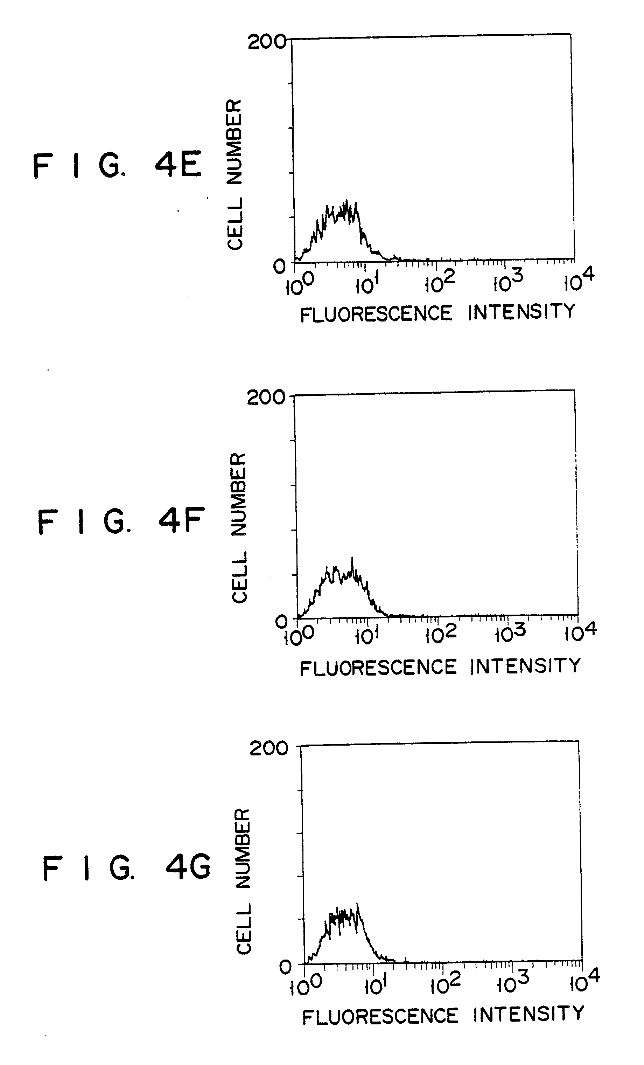

Figs. 4A-4G show results of flow cytometry of the seven mice. In each graph, the abscissa axis indicates fluorescence intensity of W6/32, and the ordinate axis indicates the number of cells corresponding to each fluorescence intensity. As apparently from the graphs, all of the seven transgenic mice had HLA-B51 antigen expressed.

Consequently, HLA-B51 gene was found to be possibly introduced into a genital organ of a mouse and to stably passed.

Production of Anti-HLA-Bw52 Monoclonal Antibodies with HLA-B51 Transgenic Mouse

According to the following operations, anti-HLA-Bw52 monoclonal antibodies can be produced by utilizing the HLA-B51 transgenic mouse obtained above.

A. Immunity to Transgenic Mouse

First, HLA-Bw52 genomic DNAs comprising base sequence showed in Figs. 1A-1G are introduced into tk-mouse L cells to obtain LBw52 cells having HLA-Bw52 alloantigen expressed thereon.

One $\times 10^7$ of the LBw52 cells and complete Freund's adjuvant are mixed, and the resultant mixture is subcutaneously injected to the HLA-B51 transgenic mouse. After that, $1 \times 10^7$ of LBw52 cells are intraperitoneally injected to the mouse every two weeks for three times for boosting. Blood is collected after one week of the last injection, and titer of anti-HLA-Bw52 antibody in serum is measured by means of cytotoxicity test. To produce anti-HLA-Bw52 monoclonal antibodies, such mice as showing at least 640 times in titer in cytotoxicity test were selected.

B. Preparation of Lymphocytes

A spleen is enucleated from the immuned HLA-B51 transgenic mouse, and put into RPMI medium containing 10% FCS. Tissues around the spleen are then completely removed, and the spleen is cut into halves with scissors. The spleen is further taken to pieces with tweezers to obtain lymphocytes in the medium. Fifteen m$\ell$ of the resultant lymphocyte suspension is put into a centrifuge tube and centrifuged at 1500 rpm for five minutes to wash. After that, the resultant supernatant solution is discarded, and 10 m$\ell$ of RPMI medium is added to the tube to suspend the lymphocytes. The number of the lymphocytes suspended is measured.

### C. Preparation of Myeloma Cells

Cultured mouse myeloma cells P3X63-Ag8.653 (ATCC No. CRL-1580) are put into a 50 m$\ell$ centrifuge tube and centrifuged at 1500 rpm for five minutes, followed by discarding of the supernatant solution. After that, a FCS-free RPMI medium is added to the tube to take the pelette to pieces, and further centrifuged at 1500 rpm for five minutes, followed by discarding of the supernatant solution. To the centrifuge tube, a FCS-free RPMI medium is further added and the number of the myeloma cells is measured.

### D. Cell Fusion

The lymphocytes and myeloma cells P3X63 Ag8.653 as prepared above are fused according to Köhler and Milstein method (Köhler and Milstein, Nature 256, pp 495-497 (1975)) as follows or other methods.

First, $1 \times 10^9$ lymphocytes and $1 \times 108$ P3X63 Ag8.653 are mixed, and the resultant mixture is centrifuged at 1500 rpm for five minutes, followed by discarding the supernatant solution to make the cells pellete. Apart from that, 1 g of PEG4000 (manufactured by Merck Co.) is put into a test tube, and the tube is capped with an aluminium cap and heated in an autoclave. After that, 1 m$\ell$ of a FCS-free RPMI medium is added to the tube, and the tube is warmed in warm bath at 37°C to prepare PEG solution. One m$\ell$ of this PEG solution is poured into the centrifuge tube containing said pellete cells, followed by stirring for one minute. To this tube, a FCS-free RPMI medium is added at 1 m$\ell$/min. for two minutes for gradual dilution, and 8 m$\ell$ of a FCS-free RPMI medium is further added for two minutes. After that, the centrifuge tube is centrifuged at 1500 rpm for five minutes, followed by discarding the supernatant solution. Thirty m$\ell$ of RPMI-HAT medium containing 10% FCS is then added, and the mixture solution is dispensed at 0.1 m$\ell$/well into a 96-well microplate. After 4-6 days of dispension, 0.1 m$\ell$ of RPMI-HAT medium containing 10% FCS is added. After that, 0.1 m$\ell$ of the

medium in each well is replaced with RPMI-HAT medium containing 10% FCS twice a week. After two weeks, 100 $\mu\ell$ of a supernatant solution in each well is taken and antibody screening is performed to the solution. After that, instead of RPMI-HAT medium containing 10% FCS, 0.1 m$\ell$ of the medium in each well replaced with HT medium twice a week.

The antibody screening is performed according to complement-dependent cytotoxicity test (Terasaki, P.I. and McCleland, J.D. Nature (London), 204, pp 998-1000, 1964). This test is to confirm that antibodies in the supernatant solution of test hybridomas combine with HLA-Bw52 antigens on human peripheral blood lymphocytes, by making the lymphocytes expressing HLA-Bw52 antigen to react with the supernatant solution. Such hybridomas as confirmed to be positive twice in the complement-dependent cytotoxicity test are transferred to a 24-well culture plate when they sufficiently grown.

Next, the hybridomas are grown in the 24-well plate, and the sufficiently prolifirated hybridomas are dispensed in a 96-well microplate at 0.6 hybridoma/well. Gamma-ray irradiated C3H tymocytes are further dispensed into each well at $1 \times 10^6$/well as feeder cells. After that, the hybridomas are cultured with a half of the medium in each well replaced with HT medium twice a week. When replacing, the supernatant solution aspirated from each well is subjected to antibody screening. Then, cloning is performed by making such hybridomas as showing positive by the antibody screening to grow. After cloning, allospecificity of the antibodies in supernatant solution of positive hybridomas is determined by utilizing panel cells.

### E. Purification of Monoclonal Antibodies

Such hybridomas as producing monoclonal antibodies whose allospecificity is determined are cultured in HT medium. The resultant supernatant solution is subjected to affinity column filled with goat anti-mouse immunoglobulin antibody combining agarose. The resultant adsorbed fraction is eluted with borate buffer containing 3M KSCN, pH 8.0, to purify monoclonal antibodies.

When the hybridomas are cultured in vivo, monoclonal antibodies are purified according to the following operation. First, $1 \times 10^7$ hybridomas are intraperitoneally injected into a C3H mouse. Next, the ascites fluid is taken from the mouse after 15-20 days of the injection. The fluid is subjected to reverse phase HPLC for gel filtration to obtain a monoclonal antibody fraction of immunoglobulin class. Anti-HLA-Bw52 monoclonal antibodies are thus obtained.

## Production of HLA-B51 Transgenic Mouse Utilizing Germ-Line Chimeras

### A. Picking of Embryos

Five units of pregnantmare serum (PMS) is intraperitoneally injected to a C3H female mouse (C3H/He, obtained from the SHIZUOKA ANIMAL CENTER) and, after the passing of 48 hours, 5 units of chorionic gonadotropin (HCG). This mouse is put in a cage in which a C3H male mouse is present. The mice are observed until the copulation is confirmed.

Two days after the confirmation of the copulation, the female mouse is killed, and the oviduct and portions of the uterus are enucleated. Embryos (between) 8-12 embryo-derived cells are picked up from the enucleated organs. The embryos are treated with hyaluronidase, and rinsed with medium. Thereafter, the embryos are cultured at 37° C in a $CO_2$ incubtor.

### B. Introduction of Genes into ES Cell

The HLA-B51 genes prepared in the above example can be introduced into an embryonal stem cell (ES Cell) by the following procedures: -

First, a medium is prepared in a culture dish. A drop of solution of HLA-B51 genomic DNA having base sequences shown in Figs. 1A to 1G is prepared, and the drop is covered with paraffin oil. Then, a picked-up ES cell (embryonal stem cell) is shifted to the drop of medium. Then, the culture dish is set on an injection microscope. The microscope has a micro manipulator which is provided with a holding pipette for fixing the cell, and an injection pipette for injecting DNA solution. After the dish is set on the microscope, the DNA solution is sucked by the injection pipette. Then, in the state wherein the ES cell is being fixed by the holding pipette, the DNA solution sucked by the injection pipette is injected into the nucleus of the ·embryonal stem cell.

### C. Injection of Transfected ES Cell in Embryos

The embryos are shifted to an injection chamber which is cooled in advance. A number of ES cells (about 1 to 15), which are sufficient for a single injection, are sucked in the injection pipette. The injection pipette is slowly thrusted into the embryos. Then, the ES cells are slowly extruded and injected.

The injected embryos are put in the oil-covered drop of DMEM + 10% FCS, and are cultured for a day at 37° C.

### D. Transfer of Embryos into Foster Mother

First, a male mouse is subjected to vasectomy and sterilized. The male mouse is made to copulate with a female ICR mouse, and the female mouse is brought into the state of pseudopregnancy. Three days after, the embryos, in which the ES cells were injected, are transferred into the female mouse in the state of pseudopregnancy in the manner stated below. First 6 mg/ml nembutal (per mouse) is intraperitoneally injected into the mouse thereby anesthetizing the same. Then, the mouse is laid so as to face downward, and the skin and muscular layer of the mouse are cut open from the back. Then, a fatty lump around the ovarium is taken out by means of a pair of tweezers. The uterine tube is found from the taken-out fatty lump, and the embryos are shifted into the uterine tube by means of a transplanting pipette. After a fertilized egg is transfered, the overium, uterine tube and uterus are restored in the abdominal cavity. The muscular layer is sutured, and the skin is put together by means of a clip. The ICR mouse which already derived is made to bring up a mouse born after bout 20 days.

### E. Confirmation of Transgenic Mouse

It is confirmed whether or not genes are transfered in the germ cells of the obtained mouse, i.e., whether HLA-B51 genes are integrated in the DNA picked up from the tail of the mouse ($F_0$ mouse) by means of the dot-blot method in E.(1) of first Example.

Next, the $F_0$ mouse is mated with a normal mouse of the same kind, thus preparing $F_1$ mice. Regarding the $F_1$ mice, the integration of the HLA-B51 genes is confirmed by means of the aforementioned dot-blot hybridization.

The $F_0$ mouse, with respect to which it is found, from the result of the dot-blot hybridization, that HLA-B51 genes were introduced in the germ cells but the HLA-B51 genes were not introduced into the somatic cell, is a chimera and not a transgenic mouse. However, if the result of the dot-blot hybridization regarding the $F_1$ mouse which was born from the $F_0$ mouse is positive, the $F_1$ mouse is a transgenic mouse.

The $F_1$ mouse, with respect to which the result of the dot-blot hybridization is positive, is subjected to the flow cytometry for testing the expression of the HLA-B51 alloantigen, which is described in E.-(2) of first Example.

The F$_1$ mouse, with respect to which the results of both the dot-blot hybridization and the flow cytometry are positive, can be used in the manufacture of the monoclonal antibody. The monoclonal antibody can be manufactured according to the method above described.

Additional advantages and modifications will readily occur to those skilled in the art. Therefore, the invention in its broader aspects is not limited to the specific details, and illustrated examples shown and described herein. Accordingly, various modifications may be made without departing from the spirit or scope of the general inventive concept as defined by the appended claims and their equivalents.

## Claims

1. A method for producing anti-HLA monoclonal antibodies comprising;

a) a step of introducing HLA gene into a fertilized egg or an embryo of non-human mammal,

b) a step of transferring said fertilized egg or said embryo of the non-human mammal obtained in the step (a) into a uterus of a female non-human mammal which is a foster mother,

c) a step of breeding said female non-human mammal of a foster mother for a given period so that the mammal may reproduce transgenic non-human mammal,

d) a step of administering HLA antigen as immunogen into said transgenic non-human mammals whose introduced HLA genes are expressed,

e) a step of enucleating the spleen from said transgenic non-human mammal immuned in the step (d) and making lymphocytes obtained from the spleen and myeloma cells to fuse,

f) a step of selecting hybridomas which produce anti-HLA antibodies among hybridomas obtained in the step of (e), and

g) a step of culturing the selected hybridomas in vivo or in vitro to obtain anti-HLA monoclonal antibodies.

2. A method for producing anti-HLA monoclonal antibodies according to claim 1, characterized in that in said step (a), HLA gene is directly injected into pronuclei in said fertilized egg.

3. A method for producing anti-HLA monoclonal antibodies comprising;

a) a step of transferring embryonal stem cells having HLA gene introduced into a uterus of female non-human mammal of a foster mother, simultaneously with an early embryo of non-human mammal,

b) a step of breeding said female non-human mammal of a foster mother for a given period so that the mammal reproduce chimera individuals having HLA genes in their germ cells,

c) a step of performing a passage of said chimera individuals obtained in the step of (b) to obtain transgenic non-human mammals having HLA genes in their all germ cells and somatic cells,

d) a step of administering HLA antigens as immunogen into transgenic non-human mammals which are obtained in the step of (c) and whose HLA genes introduced are expressed,

e) a step of enucleating a spleen from said transgenic non-human mammal immuned in the step of (d) and making lymphocytes obtained from the spleen and myeloma cells to fuse,

f) a step of selecting hybridomas which produce anti-HLA antibodies among hybridomas obtained in the step of (e), and

g) a step of culturing said hybridomas selected in the step of (f) in vivo or in vitro to obtain anti-HLA monoclonal antibodies.

4. A method for producing anti-HLA monoclonal antibodies according to claim 1 or 3, characterized in that said HLA gene is HLA class I genomic gene and the immunogen is eucaryocytes which is transformed with HLA class I genomic gene for coding alloantigen that is different from alloantigen coded by said HLA class I genomic gene.

5. A method for producing anti-HLA monoclonal antibodies according to claim 4, characterized in that said eucaryocytes are mouse L cells.

6. A method for producing anti-HLA monoclonal antibodies according to claim 1 or 3, characterized in that said HLA gene is HLA class II genomic gene and the immunogen is encaryocytes which is transformed with HLA class II genomic gene for coding alloantigen that is different from alloantigen coded by said HLA class II genomic gene.

7. A method for producing anti-HLA monoclonal antibodies according to claim 6, characterized in that said eucaryocytes are mouse L cells.

8. A method for producing anti-HLA monoclonal antibodies according to claim 1 or 3, characterized in that said non-human mammal is Rodent.

9. A method for producing anti-HLA monoclonal antibodies according to claim 8, characterized in that said Rodent is a mouse.

10. A method for producing anti-HLA monoclonal antibodies according to claim 1 or 3, characterized in that said myeloma cells are mouse myeloma cells.

11. A method for producing anti-HLA monoclonal antibodies according to claim 1 or 3, characterized in that said HLA genes are a plural HLA genes which code for a plural of allotypes of anti-

gens.

EP 0 383 183 A1

$$\text{F I G. IA} \begin{cases}\end{cases}$$

EXON-1
B51    ATGCGGGTCACGGCGCCCCGAACCGTCCTCCTGCTGCTCTGGGGGGCAGT 50
Bw52   --------------------------------------------------

B51    GGCCCTGACCGAGACCTGGGCCG 73
Bw52   -----------------------

$$\text{F I G. IE} \begin{cases}\end{cases}$$

EXON-5
B51    AGCCATCTTCCCAGTCCACCATCCCCATCGTGGGCATTGTTGCTGGCCTG 50
Bw52   --------------------------------------------------

B51    GCTGTCCTAGCAGTTGTGGTCATCGGAGCTGTGGTCGCTACTGTGATGTG 100
Bw52   --------------------------------------------------

B51    TAGGAGGAAGAGCTCAG 117
Bw52   -----------------

$$\text{F I G. IF} \begin{cases}\end{cases}$$

EXON-6
B51    GTGGAAAAGGAGGGAGCTACTCTCAGGCTGCGT 33
Bw52   ---------------------------------

$$\text{F I G. IG} \begin{cases}\end{cases}$$

EXON-7
B51    CCAGCGACAGTGCCCAGGGCTCTGATGTGTCTCTCACAGCTTGA 44
Bw52   --------------------------------------------

EXON-2(α1-DOMAIN).

B51   GCTCCCACTCCATGAGGTATTTCTACACCGCCATGTCCCGGCCCGGCCGC
Bw52  --------------------------------------------------

B51   GGGGAGCCCCGCTTCATTGCAGTGGGCTACGTGGACGACACCCAGTTCGT
Bw52  -----------------------C--------------------------

B51   GAGGTTCGACAGCGACGCCGCGAGTCCGAGGACGGAGCCCCGGGCGCCAT
Bw52  --------------------------------------------------

B51   GGATAGAGCAGGAGGGGCCGGAGTATTGGGACCGGAACACACAGATCTTC
Bw52  --------------------------------------G-G------C-

B51   AAGACCAACACACAGACTTACCGAGAGAACCTGCGGATCGCGCTCCGCTA
Bw52  --------------------------------------------------

B51   CTACAACCAGAGCGAGGCCG
Bw52  --------------------

F I G. 1B

EP 0 383 183 A1

EXON-3(α2-DOMAIN)

```
                                    •               •               •               •         50
B51    GGTCTCACACTTGGCAGACGATGTATGGCTGCGACGTGGGGCCGGACGGG
Bw52   --------------------------------------------------

              •               •               •               •       100
B51    CGCCTCCTCCGCGGGCATAACCAGTACGCCTACGACGGCAAAGATTACAT
Bw52   --------------------------------------------------

              •               •               •               •       150
B51    CGCCCTGAACGAGGACCTGAGCTCCTGGACCGCGGCGGACACCGCGGCTC
Bw52   --------------------------------------------------

                   •               •               •             •      200
B51    AGATCACCCAGCGCAAGTGGGAGGCGGCCCGTGAGGCGGAGCAGCTGAGG
Bw52   --------------------------------------------------

              •               •               •            •         250
B51    GCCTACCTGGAGGGCCTGTGCGTGGAGTGGCTCCGCAGACACCTGGAGAA
Bw52   --------------------------------------------------

              •            •    276
B51    CGGGAAGGAGACGCTGCAGGCCGCGG
Bw52   -------------------------
```

F I G. 1C

EP 0 383 183 A1

EP 0 383 183 A1

EXON-4 (α3-DOMAIN)                                                    50
B51    ACCCCCCAAAGACACACGTGACCCACCACCCCGTCTCTGACCATGAGGCC
Bw52   ──────────────────────────────────────────────────

                                                                    100
B51    ACCCTGAGGTGCTGGGCCCTGGGCTTCTACCCTGCGGAGATCACACTGAC
Bw52   ──────────────────────────────────────────────────

                                                                    150
B51    CTGGCAGCGGGATGGCGAGGACCAAACTCAGGACACTGAGCTTGTGGAGA
Bw52   ──────────────────────────────────────────────────

                                                                    200
B51    CCAGACCAGCAGGAGATAGAACCTTCCAGAAGTGGGCAGCTGTGGTGGTG
Bw52   ────────────────────────────────────────────────

                                                                    250
B51    CCTTCTGGAGAAGAGCAGAGATACACATGCCATGTACAGCATGAGGGGCT
Bw52   ──────────────────────────────────────────────────

                                            276
B51    GCCGAAGCCCCTCACCCTGAGATGGG
Bw52   ──────────────────────────

F I G. 1D

FIG. 2

EP 0 383 183 A1

F I G. 3A

F I G. 3B

F I G. 4A

F I G. 4B

F I G. 4C

F I G. 4D

F I G. 4E

F I G. 4F

F I G. 4G

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | IMMUNOBIOLOGY, vol. 178, nos. 1-2, 1988, page 122; G.J. HÄMMERLING et al.: "HLA transgenic mice as a novel tool for the generation of allele specific HLA antibodies" <br> * Whole abstract * <br> ----- | 1-11 | C 12 N 15/00 <br> C 12 P 21/08 |
|  |  |  | TECHNICAL FIELDS SEARCHED (Int. Cl.5) |
|  |  |  | C 12 N <br> C 12 P |

The present search report has been drawn up for all claims

| Place of search | Date of completion of the search | Examiner |
|---|---|---|
| THE HAGUE | 22-05-1990 | ROSDY M.K.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)